(19) Europäisches Patentamt
European Patent Office
Office européen des brevets

(11) **EP 1 578 977 B1**

(12) **EUROPÄISCHE PATENTSCHRIFT**

(45) Veröffentlichungstag und Bekanntmachung des Hinweises auf die Patenterteilung:
**17.08.2011 Patentblatt 2011/33**

(21) Anmeldenummer: **03813908.5**

(22) Anmeldetag: **23.12.2003**

(51) Int Cl.:
*C12N 15/82* (2006.01)    *C07K 14/415* (2006.01)
*A01H 5/00* (2006.01)

(86) Internationale Anmeldenummer:
**PCT/EP2003/014774**

(87) Internationale Veröffentlichungsnummer:
**WO 2004/057946 (15.07.2004 Gazette 2004/29)**

(54) **VERFAHREN ZUR VERÄNDERUNG DES GEHALTS VON SPEICHERSTOFFEN IN PFLANZEN**

METHOD FOR ALTERING THE CONTENT OF RESERVE SUBSTANCES IN PLANTS

PROCEDE DE MODIFICATION DE LA TENEUR EN RESERVES DE PLANTES

(84) Benannte Vertragsstaaten:
**AT BE BG CH CY CZ DE DK EE ES FI FR GB GR HU IE IT LI LU MC NL PT RO SE SI SK TR**

(30) Priorität: **23.12.2002 DE 10260707**

(43) Veröffentlichungstag der Anmeldung:
**28.09.2005 Patentblatt 2005/39**

(73) Patentinhaber: **Max-Planck-Gesellschaft zur Förderung der Wissenschaften e.V.
80539 München (DE)**

(72) Erfinder:
• **GEIGENBERGER, Peter
14129 Berlin (DE)**
• **LANGER, Anke
14471 Potsdam (DE)**
• **VIGEOLAS, Helene
14469 Potsdam (DE)**
• **STITT NIGEL, Marc
14469 Potsdam (DE)**
• **VAN DONGEN, Thomas Joost
14473 Potsdam (DE)**
• **UDVARDI, Michael
14476 Golm (DE)**

(56) Entgegenhaltungen:
WO-A-98/12913    WO-A-99/02687
US-B1- 6 372 961

• **BARATA R M ET AL: "Targeting of the soybean leghemoglobin to tobacco chloroplasts: Effects on aerobic metabolism in transgenic plants" PLANT SCIENCE (SHANNON), Bd. 155, Nr. 2, 29. Juni 2000 (2000-06-29), Seiten 193-202, XP002283115 ISSN: 0168-9452**
• **STOUGAARD, J. ET AL: "Expression of a complete soybean leghemoglobin gene in root nodules of transgenic Lotus corniculatus." PROCEEDINGS OF THE NATIONAL ACADEMY OF SCIENCES OF THE UNITED STATES OF AMERICA, PUBLISHER: WASHINGTON, D.C.: THE ACADEMY. CODEN: PNASA6;ISSN: 0027-8424, Bd. 84, Nr. 16, 1987, Seiten 5754-5757, XP002283117**
• **DATABASE EMBL [Online] EBI, Hinxton; 18. November 2000 (2000-11-18), UCHIUMI T ET AL: "Genomic leghemoglobin genes from Lotus japonicus" XP002283119 Database accession no. AB042718**
• **DATABASE EMBL [Online] EBI, Hinxton; 6. November 1997 (1997-11-06), TREVASKIS B ET AL: "Two hemoglobin genes in Arabidopsis thaliana: The evolutionary origins of leghemoglobins" XP002283120 Database accession no. U94999 -& TREVASKIS B ET AL: PROCEEDINGS OF THE NATIONAL ACADEMY OF SCIENCES OF THE UNITED STATES OF AMERICA PUBLISHER: WASHINGTON, D.C.: NATIONAL ACADEMY OF SCIENCES, CODEN: PNASA6;ISSN: 0027-8424, Bd. 94, Oktober 1997 (1997-10), Seiten 12230-12234, XP002283114**

SEQUENCE LISTING

<110> Max-Planck-Gesellschaft

<120> Verfahren zur Veränderung des Gehalts von Speicherstoffen in Pflanzen

<130> NAE 737/02 PCT

<150> DE 10260707.9
<151> 2002-12-23

<160> 6

<170> PatentIn version 3.2

<210> 1
<211> 444
<212> DNA

<213> Lotus japonicus

<220>
<221> CDS
<222> (1)..(444)

<400> 1

```
atg ggt ttc act gcg cag caa gag gct cta gtg ggt agc tca tac gaa    48
Met Gly Phe Thr Ala Gln Gln Glu Ala Leu Val Gly Ser Ser Tyr Glu
1               5                   10                  15


aca ttc aag aaa aac ctt cct acc aac agt gtt ttg ttc tac acc gtt    96
Thr Phe Lys Lys Asn Leu Pro Thr Asn Ser Val Leu Phe Tyr Thr Val
```

|  | 20 |  | 25 |  | 30 |  |

ata ttg gag ata gca cca act gca aaa gac atg ttc tcc ttt cta aag
144
Ile Leu Glu Ile Ala Pro Thr Ala Lys Asp Met Phe Ser Phe Leu Lys
        35            40            45

gag tct ggg cct aag cat agt cct cag ctc cag gcc cat gct gaa aag
192
Glu Ser Gly Pro Lys His Ser Pro Gln Leu Gln Ala His Ala Glu Lys
        50            55            60

gtt ttt gca ctg act cgt gat gct gcc act caa ctc gta gca aaa gga
240
Val Phe Ala Leu Thr Arg Asp Ala Ala Thr Gln Leu Val Ala Lys Gly
65            70            75            80

gaa gtg aca ctt gca gat gcc agc tta ggt gct gtc cac gtt cag aaa
288
Glu Val Thr Leu Ala Asp Ala Ser Leu Gly Ala Val His Val Gln Lys
          85           90           95

gcc gtt act gat cct cat ttc gtg gtg gtt aaa gaa gcc ctg ctt caa
336
Ala Val Thr Asp Pro His Phe Val Val Val Lys Glu Ala Leu Leu Gln
          100         105         110

aca gta aag gaa gca gtt ggg gcg gac gaa tgg agt gat gac ttg agc
384
Thr Val Lys Glu Ala Val Gly Ala Asp Glu Trp Ser Asp Asp Leu Ser
          115         120         125

acc gct tgg gaa gga gca tat gat gga cta gca act gca att aag aag
432
Thr Ala Trp Glu Gly Ala Tyr Asp Gly Leu Ala Thr Ala Ile Lys Lys

                    130                    135                    140

gca atg ggt taa
444
Ala Met Gly
145


<210> 2
<211> 147
<212> PRT
<213> Lotus japonicus

<400> 2


Met Gly Phe Thr Ala Gln Gln Glu Ala Leu Val Gly Ser Ser Tyr Glu
1               5                   10                  15


Thr Phe Lys Lys Asn Leu Pro Thr Asn Ser Val Leu Phe Tyr Thr Val
            20                  25                  30


Ile Leu Glu Ile Ala Pro Thr Ala Lys Asp Met Phe Ser Phe Leu Lys
            35                  40                  45


Glu Ser Gly Pro Lys His Ser Pro Gln Leu Gln Ala His Ala Glu Lys
            50                  55                  60


Val Phe Ala Leu Thr Arg Asp Ala Ala Thr Gln Leu Val Ala Lys Gly
65                  70                  75                  80

```
Glu Val Thr Leu Ala Asp Ala Ser Leu Gly Ala Val His Val Gln Lys
                    85                  90                  95

Ala Val Thr Asp Pro His Phe Val Val Val Lys Glu Ala Leu Leu Gln
                    100                 105                 110

Thr Val Lys Glu Ala Val Gly Ala Asp Glu Trp Ser Asp Asp Leu Ser
                115                 120                 125

Thr Ala Trp Glu Gly Ala Tyr Asp Gly Leu Ala Thr Ala Ile Lys Lys
            130                 135                 140

Ala Met Gly
145
```

<210> 3
<211> 483
<212> DNA
<213> Arabidopsis thaliana

<220>
<221> CDS
<222> (1)..(483)

<400> 3

```
atg gag agt gaa gga aag att gtg ttc aca gaa gag caa gag gct ctt     48
Met Glu Ser Glu Gly Lys Ile Val Phe Thr Glu Glu Gln Glu Ala Leu
1               5                   10                  15
```

gta gtg aag tct tgg agt gtc atg aag aaa aac tca gct gaa tta ggt

96

Val Val Lys Ser Trp Ser Val Met Lys Lys Asn Ser Ala Glu Leu Gly
          20                  25              30

ctc aaa ctc ttc atc aag atc ttt gag att gca cca aca acg aag aag

144

Leu Lys Leu Phe Ile Lys Ile Phe Glu Ile Ala Pro Thr Thr Lys Lys
              35                  40                  45

atg ttc tct ttc ttg aga gac tca cca att cct gct gag caa aat cca

192

Met Phe Ser Phe Leu Arg Asp Ser Pro Ile Pro Ala Glu Gln Asn Pro
          50                  55                  60

aag ctc aag cct cac gca atg tct gtt ttt gtc atg tgt tgt gaa tca

240

Lys Leu Lys Pro His Ala Met Ser Val Phe Val Met Cys Cys Glu Ser
65                  70                  75                  80

gca gta caa ctg agg aaa aca ggg aaa gtt acg gtg agg gag act act

288

Ala Val Gln Leu Arg Lys Thr Gly Lys Val Thr Val Arg Glu Thr Thr
              85                  90                  95

ttg aag aga ctt gga gcc agc cat tct aaa tac ggt gtc gtt gac gaa

336

Leu Lys Arg Leu Gly Ala Ser His Ser Lys Tyr Gly Val Val Asp Glu
              100                 105                 110

cac ttt gag gtg gcc aag tat gca ttg ttg gag acg ata aag gag gca

384

His Phe Glu Val Ala Lys Tyr Ala Leu Leu Glu Thr Ile Lys Glu Ala
              115                 120                 125

```
gtg ccg gag atg tgg tca ccg gag atg aag gtg gct tgg ggt cag gct
432
Val Pro Glu Met Trp Ser Pro Glu Met Lys Val Ala Trp Gly Gln Ala
    130                 135                 140

tat gat cac ctt gtt gct gcc att aaa gct gaa atg aat ctt tcc aac
480
Tyr Asp His Leu Val Ala Ala Ile Lys Ala Glu Met Asn Leu Ser Asn
145                 150                 155                 160

taa
483
```

<210> 4
<211> 160
<212> PRT
<213> Arabidopsis thaliana

<400> 4

```
Met Glu Ser Glu Gly Lys Ile Val Phe Thr Glu Glu Gln Glu Ala Leu
1               5                   10                  15

Val Val Lys Ser Trp Ser Val Met Lys Lys Asn Ser Ala Glu Leu Gly
            20                  25                  30

Leu Lys Leu Phe Ile Lys Ile Phe Glu Ile Ala Pro Thr Thr Lys Lys
            35                  40                  45

Met Phe Ser Phe Leu Arg Asp Ser Pro Ile Pro Ala Glu Gln Asn Pro
```

| | 50 | | | 55 | | | 60 | |
|---|---|---|---|---|---|---|---|---|

Lys Leu Lys Pro His Ala Met Ser Val Phe Val Met Cys Cys Glu Ser
65                    70                    75                    80

Ala Val Gln Leu Arg Lys Thr Gly Lys Val Thr Val Arg Glu Thr Thr
                 85                    90                    95

Leu Lys Arg Leu Gly Ala Ser His Ser Lys Tyr Gly Val Val Asp Glu
                     100                    105                    110

His Phe Glu Val Ala Lys Tyr Ala Leu Leu Glu Thr Ile Lys Glu Ala
                 115                    120                    125

Val Pro Glu Met Trp Ser Pro Glu Met Lys Val Ala Trp Gly Gln Ala
                 130                    135                    140

Tyr Asp His Leu Val Ala Ala Ile Lys Ala Glu Met Asn Leu Ser Asn
145                    150                    155                    160

<210> 5
<211> 477
<212> DNA
<213> Arabidopsis thaliana

<220>
<221> CDS
<222> (1)..(477)

<400> 5

EP 1 578 977 B1

```
atg gga gag att ggg ttt aca gag aag caa gaa gct ttg gtg aag gaa    48
Met Gly Glu Ile Gly Phe Thr Glu Lys Gln Glu Ala Leu Val Lys Glu
1               5                   10                  15


tcg tgg gag ata ctg aaa caa gac atc ccc aaa tac agc ctt cac ttc    96
Ser Trp Glu Ile Leu Lys Gln Asp Ile Pro Lys Tyr Ser Leu His Phe
                20                  25                  30


ttc tca cag ata ctg gag ata gca cca gca gca aaa ggc ttg ttc tct   144
Phe Ser Gln Ile Leu Glu Ile Ala Pro Ala Ala Lys Gly Leu Phe Ser
            35                  40                  45


ttc cta aga gac tca gat gaa gtc cct cac aac aat cct aaa ctc aaa   192
Phe Leu Arg Asp Ser Asp Glu Val Pro His Asn Asn Pro Lys Leu Lys
        50                  55                  60


gct cat gct gtt aaa gtc ttc aag atg aca tgt gaa aca gct ata cag   240
Ala His Ala Val Lys Val Phe Lys Met Thr Cys Glu Thr Ala Ile Gln
65                  70                  75                  80


ctg agg gag gaa gga aag gtg gta gtg gct gac aca acc ctc caa tat   288
Leu Arg Glu Glu Gly Lys Val Val Val Ala Asp Thr Thr Leu Gln Tyr
                85                  90                  95


tta ggc tca att cat ctc aaa agc ggc gtt att gac cct cac ttc gag   336
```

```
Leu Gly Ser Ile His Leu Lys Ser Gly Val Ile Asp Pro His Phe Glu
            100             105                 110

gtg gtg aaa gaa gct ttg cta agg aca ttg aaa gag ggg ttg ggg gag
384
Val Val Lys Glu Ala Leu Leu Arg Thr Leu Lys Glu Gly Leu Gly Glu
        115                 120                 125

aaa tac aat gaa gaa gtg gaa ggt gct tgg tct caa gct tat gat cac
432
Lys Tyr Asn Glu Glu Val Glu Gly Ala Trp Ser Gln Ala Tyr Asp His
        130                 135                 140

ttg gct tta gcc atc aag acc gag atg aaa caa gaa gag tca taa
477
Leu Ala Leu Ala Ile Lys Thr Glu Met Lys Gln Glu Glu Ser
145                 150                 155
```

<210> 6
<211> 158
<212> PRT
<213> Arabidopsis thaliana

<400> 6

```
Met Gly Glu Ile Gly Phe Thr Glu Lys Gln Glu Ala Leu Val Lys Glu
1               5               10              15

Ser Trp Glu Ile Leu Lys Gln Asp Ile Pro Lys Tyr Ser Leu His Phe
            20              25              30

Phe Ser Gln Ile Leu Glu Ile Ala Pro Ala Ala Lys Gly Leu Phe Ser
```

```
                35                    40                    45


    Phe Leu Arg Asp Ser Asp Glu Val Pro His Asn Asn Pro Lys Leu Lys
        50              55              60


    Ala His Ala Val Lys Val Phe Lys Met Thr Cys Glu Thr Ala Ile Gln
    65              70              75              80


    Leu Arg Glu Glu Gly Lys Val Val Val Ala Asp Thr Thr Leu Gln Tyr
                    85              90              95


    Leu Gly Ser Ile His Leu Lys Ser Gly Val Ile Asp Pro His Phe Glu
                100             105             110


    Val Val Lys Glu Ala Leu Leu Arg Thr Leu Lys Glu Gly Leu Gly Glu
                115             120             125


    Lys Tyr Asn Glu Glu Val Glu Gly Ala Trp Ser Gln Ala Tyr Asp His
        130             135             140


    Leu Ala Leu Ala Ile Lys Thr Glu Met Lys Gln Glu Glu Ser
    145             150             155
```

## Beschreibung

Die Erfindung betrifft ein Verfahren zur Veränderung des Gehalts von Speicherstoffen in Pflanzen durch Einsatz von Leghemoglobin und/oder Hemoglobin exprimierender transformierter Pflanzen, entsprechende Pflanzen sowie deren Verwendung.

Speicherstoffe in Pflanzen dienen als Reservestoffe und werden von pflanzlichen Geweben gebildet und intrazellulär abgelagert. Als Speicherstoffe dienen z. B. Polysaccharide (Kohlenhydrate wie Lichenin, Stärke, Glykogen, Polyfructosane), Proteine, Fette, auch Polyphosphate und Polyhydroxyalkanoate. Die Speicherstoffe können bei Bedarf wieder in den Stoff- und Energiewechsel eingeschleust werden, beispielsweise bei Nahrungsmangel, der Keimung von Samen, dem Wachstum und sonstigen energieverbrauchenden Vorgängen.

Speicherstoffe sind wertvolle Rohstoffe für die Ernährung (Getreide, Nüsse, Früchte, Obst, Gewürze etc.) und bilden die Basis vieler menschlicher Nahrungsmittel und können auch als Lieferanten für technische Fette und Öle dienen. Andere Speicherstoffe werden wegen ihrer pharmakologisch wirksamen Inhaltsstoffe arzneilich genutzt. (Quelle: CD Römpp Chemie Lexikon - Version 1.0, Stuttgart/New York: Georg Thieme Verlag 1995).

Auch werden in zunehmendem Maße die Speicherstoffe als nachwachsende und ökologisch verträgliche Rohstoffe für die Industrie angewendet, wie dies z. B. durch die Verwendung von Stärke zur Herstellung von Verpackungsmaterialien oder z. B. von Pflanzenölen als Kraftstoffe, wie Biodiesel oder Schmierstoffe möglich ist.

Die sogenannten sekundären Stoffwechselprodukte (sek. Metaboliten, vgl. a. Stoffwechsel) von Pflanzen (z. B. Farbstoffe, Gifte, etherische Öle, Alkaloide, Fruchtsäuren) werden im allg. nicht zu den Speicherstoffen gezählt (Quelle: Römpp Lexikon Chemie - Version 2.0, Stuttgart/New York: Georg Thieme Verlag 1999 "Reservestoffe").

In Pflanzen werden die Speicherstoffe in Samen oder Speicherorganen von Kohlenhydrat-Vorstufen gebildet. Dabei ist Saccharose die Primärquelle von Kohlenstoff und Energie, welche von den Blättern in die sich entwickelnden Samen bzw. in Speicherorgane transportiert wird. Die Saccharose in den Blättern wird dabei durch die aus der Photosynthese gewonnene Stärke gebildet. Die Saccharose, die in die sich entwickelnden Samen transportiert wird, dient neben der Synthese von Fettsäuren für die Speicherlipide auch zur Synthese von Speicherproteinen und Speicherstärke.

Insgesamt enthalten z. B. Samen drei verschiedene Formen an Speicherstoffen: Speicherlipide, Speicherproteine und Stärke. Je nach Pflanzen variieren dabei die Verhältnisse der drei Speicherstoffe zueinander. So enthalten Rapssorten z. B. etwa 48 % Speicherlipide, 19 % Stärke und 21 % Speicherproteine, während Sojabohne 22 % Lipide, 12 % Stärke und 37 % Proteine enthält (Biochemistry & Molecular Biology of the Plant ed. Buchanan, Gruissem, Jones 2000, American Society of Plant Physiologists) bezogen auf ihre Trockenmasse.

Die aus den pflanzlichen Ölen (Lipide) erhältlichen Fettsäuren sind von besonderem Interesse. Sie kommen beispielsweise als Grundstoffe für Weichmacher, Schmierstoffe, Tenside, Kosmetika usw. zum Einsatz oder werden in der Lebens- und Futtermittelindustrie als wertvolle Grundstoffe eingesetzt. So ist beispielsweise die Bereitstellung von Rapsölen mit Fettsäuren mittlerer Kettenlänge von besonderem Interesse, da diese besonders für die Tensidherstellung begehrt sind.

Durch die gezielte Modulation pflanzlicher Stoffwechselwege mittels gentechnischer Verfahren kann der pflanzliche Metabolismus in einer Weise vorteilhaft verändert werden, die durch klassische Züchtungsmethoden nur über langwierige Schritte bzw. überhaupt nicht zu erreichen wären. So werden z. B. ungewöhnliche Fettsäuren, beispielsweise bestimmte mehrfach ungesättigte Fettsäuren, nur in bestimmten Pflanzen bzw. überhaupt nicht in Pflanzen synthetisiert und können deshalb nur durch Expression des entsprechenden Enzyms in transgenen Pflanzen gezielt hergestellt werden (z. B. Millar et al. (2000) Trends Plant Sci 5:95-101).

Stärke als Speicherstoff kann nicht nur in Samen eingelagert werden, sondern auch in anderen Speicherorganen. Wichtige Speicherorgane für Stärke sind dabei Hypokotyl und Wurzeln. Durch Vermehrung und Vergrößerung der Zellen des Rindenparenchyms entstehen Wurzelknollen, wie z. B. Kartoffelknollen oder bei Verdickung des Wurzelhalses Wurzelrüben, wie z. B. Zuckerrübe oder Yams.

So ist Stärke z. B. der Hauptbestandteil der Kartoffel-Trockensubstanz. Neben der Verwendung als Lebensmittel dient die Kartoffel daher auch als Futtermittel und als Rohstoff zur Gewinnung von Stärke und Alkohol. Weltweit wurden 1988 269,7 Mio. t Kartoffeln geerntet. (Quelle: CD Römpp Chemie Lexikon - Version 1.0, Stuttgart/New York: Georg Thieme Verlag 1995).

Die Patente US 6,372,961, WO98/12913 und WO00/00597 beschreiben die Nutzung von Hemoglobin oder strukturverwandter Proteine (Myoglobin, bakterielles Hemoglobin) zur Erhöhung der Sauerstoffassimilation in Pflanzen. Damit soll die Keimung bzw. der Energiehaushalt der entsprechend transgenen Pflanzen verbessert sein auch unter Sauerstoffmangelbedingungen ein normales Wachstum gewährleistet werden. Auch sollen die Menge produzierter sekundärer Metabolite erhöht werden (WO98/12913 Seite 6, Zeile 24). Die Steigerung der Produktion von Speicherstoffen geht aus diesen Schriften nicht hervor.

Aufgabe der vorliegenden Erfindung war es daher, den Gehalt von Speicherstoffen in Speicherorganen von Pflanzen zu erhöhen, um somit eine bessere Nutzung von Anbauflächen, Dünger usw. und eine bessere Ausbeute (oder Ertrag) mittels der Pflanzen zu erwirtschaften. Insbesondere soll die Produktion von Öl verbessert bzw. ermöglicht werden.

Diese Aufgabe wird dadurch gelöst, daß im erfindungsgemäßen Verfahren zur Veränderung des Gehalts von Speicherstoffen in Pflanzen Leghemoglobin exprimierende transformierte Pflanzen eingesetzt werden. Die Aufgabe wird ebenfalls durch die Verwendung von Leghemoglobin exprimierende transformierte Pflanzen gelöst.

Überraschenderweise konnte festgestellt werden, dass durch Expression eines Leghemoglobins Speicherstoff-haltige transformierte Pflanzen produziert werden, die auf Grund ihres (höheren) Gehalts von Speicherstoffen eine bessere Nutzung von Anbauflächen, Dünger usw. erlauben und daher einen besseren Ertrag der Speicherstoffe, insbesondere Öl erlauben. Somit ist eine wirtschaftlich interessante Verwendung der erfindungsgemäßen Pflanzen möglich.

Das Leghemoglobin gehört zur Familie der Hemoglobin-Proteine, deren Funktion die reversible Sauerstoffbindung und Versorgung ist. Es stammt aus Wurzelknöllchen von Hülsenfrüchten (Leguminosen) und ist eine isolierbare rote Substanz, die dem Myoglobin der Wirbeltiere ähnelt. Durch die reversible Bindung von $O_2$ kann Leghemoglobin den hohen Sauerstoff-Bedarf bei der Stickstoff-Fixierung durch die Knöllchenbakterien gewährleisten. Das Apoprotein wird von den Pflanzenzellen und das Häm von den Bakterien gebildet (Quelle: CD Römpp Chemie Lexikon - Version 1.0, Stuttgart/New York: Georg Thieme Verlag 1995).

In einer weiteren bevorzugten Variante der Erfindung werden in dem erfindungsgemäßen Verfahren zur Veränderung des Gehalts von Speicherstoffen in Pflanzen Hemoglobin oder Leghemoglobin und Hemoglobin exprimierende transformierte Pflanzen eingesetzt. Demgemäß ist Gegenstand der Erfindung auch noch die Verwendung von Leghemoglobin und/oder Hemoglobin exprimierende transformierte Pflanzen.

Unter Hemoglobin werden erfindungsgemäß Eisen-II-Komplexe des Protoporphyrins verstanden.

Unter Expression wird in der vorliegenden Anmeldung die Übertragung einer genetischen Information ausgehend von DNA oder RNA in ein Genprodukt (Polypeptid oder Protein, hier Leghemoglobin oder Hemoglobin) verstanden und soll auch den Begriff der Überexpression beinhalten, womit eine verstärkte Expression gemeint ist, so dass das Fremdprotein oder das natürlich vorkommende Protein verstärkt produziert werden oder einen großen Teil des gesamten Protein-Gehaltes der Wirtszelle ausmachen.

Unter Transformation wird die Übertragung einer genetischen Information in einen Organismus, insbesondere Pflanze verstanden. Darunter sollen alle dem Fachmann bekannten Möglichkeiten zur Einschleusung der Information fallen, z. B. Mikroinjektion, Elektroporation, Teilchenbeschuss (Partikelbombardement), Agrobakterien oder Chemikalien vermittelte Aufnahme (z. B. Polyethylenglycol-vermittelter DNA-Aufnahme oder über die Siliziumcarbonatfaser-Technik). Die genetische Information kann z. B. als DNA, RNA, Plasmid und auf sonstige Weise in die Zellen eingebracht und entweder durch Rekombination ins Wirtsgenom eingebaut, in freier Form oder unabhängig als Plasmid vorliegen. Eine transformierte Pflanze ist also eine gentechnisch veränderte Pflanze.

Unter Speicherstoffe werden Polysaccharide, vorzugsweise Kohlenhydrate, Proteine, Fette, Polyphosphate und Polyhydroxyalkanoate, besonders bevorzugt Lichenin, Stärke, Glykogen, Polyfructosane verstanden.

Unter den aufgezählten Verbindungen werden Kohlenhydrate und Fette ganz besonders bevorzugt. Höchst bevorzugt als Speicherstoffe sind Stärke und Öl.

Stärke ist dem Fachmann bekannt und es wird für weitere Informationen auf das Römpp Chemie Lexikon - CD Version 2.0, Stuttgart/New York: Georg Thieme Verlag 1999, verwiesen.

"Öl" umfasst im Sinne der Erfindung neutrale und/oder polare Lipide und Mischungen derselben. Beispielhaft jedoch nicht einschränkend seien die in Tabelle 1 aufgeführten zu nennen.

Tabelle 1: Pflanzliche Lipidklassen

| Neutrale Lipide | Triacylglycerol (TAG) |
|---|---|
|  | Diacylglycerol (DAG) |
|  | Monoacylglycerol (MAG) |
|  |  |
| Polare Lipide | Monogalactosyldiacylglycerol (MGDG) |
|  | Digalactosyldiacylglycerol (DGDG) |
|  | Phosphatidylglycerol (PG) |
|  | Phosphatidylcholin (PC) |
|  | Phosphatidylethanolamin (PE) |
|  | Phosphatidylinositol (PI) |
|  | Phosphatidylserin (PS) |

(fortgesetzt)

| Sulfoquinovosyldiacylglycerol |
| --- |

Neutrale Lipide meint bevorzugt Triacylglyceride. Sowohl die neutralen als auch die polaren Lipide können ein breites Spektrum an verschiedenen Fettsäuren enthalten. Beispielhaft jedoch nicht einschränkend seien die in Tabelle 2 aufgeführten Fettsäuren zu nennen.

Tabelle 2: Übersicht über verschiedene Fettsäuren (Auswahl) [1] Kettenlänge:Anzahl der Doppelbindungen * nicht natürlicherweise in Pflanzen vorkommend

| Nomenklatur' | Name |
| --- | --- |
| 16:0 | Palmitinsäure |
| 16:1 | Palmitoleinsäure |
| 16:3 | Roughaninsäure |
| 18:0 | Stearinsäure |
| 18:1 | Ölsäure |
| 18:2 | Linolsäure |
| 18:3 | Linolensäure |
| γ-18:3 | Gamma-Linolensäure* |
| 20:0 | Arachidinsäure |
| 22:6 | Docosahexanonsäure (DHA) * |
| 20:2 | Eicosadienonsäure |
| 20:4 | Arachidonsäure (AA) * |
| 20:5 | Eicosapentaenosäure (EPA) * |
| 22:1 | Erucasäure |

Für weitergehende Informationen wird ebenfalls auf das Römpp Chemie Lexikon - CD Version 2.0, Stuttgart/New York: Georg Thieme Verlag 1999 verwiesen.

Erfindungsgemäß eignen sich alle Pflanzen zur Durchführung des erfindungsgemäßen Verfahrens.

"Pflanze" umfasst alle einjährigen und mehrjährige, monokotyledonen und dikotyledonen Pflanzen und schließt beispielhaft jedoch nicht einschränkend solche der Gattungen Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solarium, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Browaalia, Glycine, Pisum, Phaseolus, Lolium, Oryza, Zea, Avena, Hordeum, Secale, Triticum, Sorghum, Picea und Populus ein.

Bevorzugt sind Pflanzen nachfolgender Pflanzenfamilien: Amaranthaceae, Asteraceae, Brassicacea, Carophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae, Umbelliferae.

Bevorzugte monokotyle Pflanzen sind insbesondere ausgewählt aus den monokotylen Kulturpflanzen, wie zum Beispiel der Familie der Gramineae wie Reis, Mais, Weizen oder andere Getreidearten wie Gerste, Hirse, Roggen, Triticale oder Hafer sowie dem Zuckerrohr sowie alle Arten von Gräsern. Die Erfindung wird ganz besonders bevorzugt aus dikotyledone pflanzliche Organismen angewendet. Bevorzugte dikotyle Pflanzen sind insbesondere ausgewählt aus den dikotylen Kulturpflanzen, wie zum Beispiel

- Asteraceae wie Sonnenblume, Tagetes oder Calendula und andere mehr,

- Compositae, besonders die Gattung Lactuca, ganz besonders die Art sativa (Salat) und andere mehr,

- Cruciferae, besonders die Gattung Brassica, ganz besonders die Arten napus (Raps), campestris (Rübe), oleracea

4

cv Tastie (Kohl), oleracea cv Snowball Y (Blumenkohl) und oleracea cv Emperor (Broccoli) und weitere Kohlarten; und der Gattung Arabidopsis, ganz besonders die Art thaliana sowie Kresse oder Canola und andere mehr,

- Cucurbitaceae wie Melone, Kürbis oder Zucchini und andere mehr,

- Leguminosae besonders die Gattung Glycine, ganz besonders die Art max (Sojabohne) Soja sowie Alfalfa, Erbse, Bohnengewächsen oder Erdnuss und andere mehr

- Rubiaceae, bevorzugt der Unterklasse Lamiidae wie beispielsweise Coffea arabica oder Coffea liberica (Kaffeestrauch) und andere mehr,

- Solanaceae besonders die Gattung Lycopersicon, ganz besonders die Art esculentum (Tomate) und die Gattung Solanum, ganz besonders die Art tuberosum (Kartoffel) und melongena (Aubergine) sowie Tabak oder Paprika und andere mehr,

- Sterculiaceae, bevorzugt der Unterklasse Dilleniidae wie beispielsweise Theobroma cacao (Kakaostrauch) und andere mehr,

- Theaceae, bevorzugt der Unterklasse Dilleniidae wie beispielsweise Camellia sinensis oder Thea sinensis (Teestrauch) und andere mehr,

- Umbelliferae, besonders die Gattung Daucus (ganz besonders die Art carota (Karrotte)) und Apium (ganz besonders die Art graveolens dulce (Selarie)) und andere mehr; und die Gattung Capsicum, ganz besonders die Art annum (Pfeffer) und andere mehr,

sowie Lein, Soja, Baumwolle, Hanf, Flachs, Gurke, Spinat, Möhre, Zuckerrübe und den verschiedenen Baum-, Nuss- und Weinarten, insbesondere Banane und Kiwi.

Umfasst sind ferner Schmuckpflanzen, Nutz- oder Zierbäume, Blumen, Schnittblumen, Sträucher oder Rasen. Beispielhaft aber nicht einschränkend seien zu nennen Angiospermen, Bryophyten wie zum Beispiel Hepaticae (Leberblümchen) und Musci (Moose); Pteridophyten wie Farne, Schachtelhalm und Lycopoden; Gymnospermen wie Koniferen, Cycaden, Ginkgo und Gnetalen, die Familien der Rosaceae wie Rose, Ericaceae wie Rhododendrons und Azaleen, Euphorbiaceae wie Weihnachtssteme und Kroton, Caryophyllaceae wie Nelken, Solanaceae wie Petunien, Gesneriaceae wie das Usambaraveilchen, Balsaminaceae wie das Springkraut, Orchidaceae wie Orchideen, Iridaceae wie Gladiolen, Iris, Freesie und Krokus, Compositae wie Ringelblume, Geraniaceae wie Geranien, Liliaceae wie der Drachenbaum, Moraceae wie Ficus, Araceae wie Philodendron und andere mehr.

Pflanzliche Organismen im Sinne der Erfindung sind weiterhin weitere photosynthetisch aktive befähigte Organismen, wie zum Beispiel Algen, Cyanobakterien sowie Moose. Bevorzugte Algen sind Grünalgen, wie beispielsweise Algen der Gattung Haematococcus, Phaedactylum tricornatum, Volvox oder Dunaliella. Insbesondere bevorzugt ist Synechocystis.

Am meisten bevorzugt sind Ölpflanzen, d.h. Pflanzen, die bereits natürlicherweise einen hohen Ölgehalt aufweisen und/oder zu industriellen Gewinnung von Ölen verwendet werden. Diese Pflanzen können einen hohen Ölgehaltr und/oder aber eine besondere, industriell interessante Fettsäurezusammensetzung aufweisen. Bevorzugt sind Pflanzen, die einen Lipidanteil von mindestens 1 Gew.% aufweisen. Ölpflanzen umfassen beispielhaft: Bovago oficinalis (Borretsch); Brassica Arten wie *B. campestris*, *B. napus*, *B. rapa* (Senf oder Raps); *Cannabis sativa* (Hanf); Curthamus *tinctorius* (Färberdiestel); Cocos *nucifera* (Kokusnuss); Crambe abyssinica (Krambe); Cuphea Arten (Cuphea Arten liefern fettsäuren von mittlerer Kettenlänge insbesondere für industrielle Anwendungen); *Elaeis guinensis* (Afrikanische Ölpalme); *Ekeis oleiferu* (Amerikanische Ölpalme); *Glycine max* (Sojabohne); *Gossypium hirisfum* (Amerikanische Baumwolle); *Gossypium barbadense* (Ägyptische Baumwolle); *Gossypium herbaceum* (Asiatische Baumwolle Cotton ); *Helianthus annus* (Sonnenblume); *Linum usitatissimum* (Lein oder Flachs); *Oenethem biennis* (Evening primrose); Ozea *europea* (Olive); Oryza *sativa* (Rice); *Ricinus communis* (Castor); Sesamum *indicum* (Sesam); *Glycine max* (Sojabohne); *Triticum* Arten (Weizen); Zea *maize* (Mais) sowie verschiedene Nussarten wie beispielsweise Walnuss oder Mandel.

Wenn es sich bei den verwendeten Pflanzen um solche zur Gattung der Leguminosen (Hülsenfrüchte) gehörenden Pflanzen handelt, so fällt unter die Erfindung die Expression von Fremdproteinen (Leghemoglobinen, Hemoglobin), d. h. in der Natur nicht-symbiontisch vorkommenden Leghemoglobinen und/oder Hemoglobinen oder die Veränderung der Pflanzen derart, dass sie das natürlich vorkommende Leghemoglobin überexprimieren.

Höchst bevorzugt sind Kartoffeln, Arabidopsis thaliana und Raps.

Vorteilhaft ist es, wenn die vorgenannten Pflanzen ein Leghemoglobin und/oder Hemoglobin ausgewählt aus der Gruppe bestehend aus Leghemoglobin und/oder Hemoglobin aus den Pflanzen Lupinus luteus (LGB1_LUPLU, LGB2_LUPLU), Glycine max (LGBA_SOYBN, LGB2_SOYBN, LGB3_SOYBN), Medicago sativa (LGB1-4_MEDSA), Medicago trunculata (LGB1_MEDTR), Phaseolus vulgaris (LGB1_PHAVU, LGB2_PHAVU), Vicia faba (LGB1_VICFA, LGB2_VICFA), Pisum sativum (LGB1_PEA, LGB2_PEA), Vigna unguiculata (LGB1_VIGUN), Lotus japonicus (LGB_ LOTJA), Psophocarpus tetragonolobus (LGB_PSOTE), Sesbania rostrata (LGB1_SESRO), Casuarina glauca (HBPA_ CASGL) und Canvalaria lineata (HBP_CANLI) Physcomitrella patens (HBL0_PHYPA), Arabidopsis thaliana (HBL1_ARATH, HBL2_ARATH), Gossypium hirsutum (HBL1-GOSHI, HBL2_GOSHI), Medicago sativa (HBL1_MEDSA), Oryza sativa (HBL1_ORYSA, HBL2_ORYSA, HBL3_ORYSA, HBL4_ORYSA), Brassica napus (HBL2_BRANA), Lycopersicon esculentum (HBL2_LYCES), Hordeum vulgare (HBL_HORVU), Zea mays (HBL_MAIZE), Trema tomentosa (HBL_TRETO), Casuarina glauca (HBP1_CASGL, HBP2_CASGL, HBPA_CASGL), Parasponia rigida (HBPL_PARAD) exprimieren. In Klammern sind die jeweiligen Swiss-Prot Datenbankeinträge vermerkt.

Besonders vorteilhaft sind Pflanzen, die die Sequenz Nr. 1 codierend für Leghemoglobin und/oder die Sequenzen Nr. 3 und/oder 5 codierend für Hemoglobin aufweisen. Bevorzugterweise wird Leghemoglobin und/oder Hemoglobin aus *Lotus japonicus* eingesetzt. Dann produzieren die transformierten Pflanzen Speicherstoffe in erhöhtem Ausmaß. Beschrieben werden Pflanzen, die zur Produktion von Speicherstoffen ein Leghemoglobin, gemäß Sequenz Nr. 1 und/ oder ein Hemoglobin gemäß Sequenzen Nr. 3 und 5 exprimieren.

In einer bevorzugten Variante der Erfindung handelt es sich um Pflanzen, die das Leghemoglobin und/oder Hemoglobin speicherorganspezifisch exprimieren.

Die genannten Pflanzen lagern die Speicherstoffe in der Regel in bestimmten Organen ab. Dies sind z. B. Zwiebeln, Knollen, Samen, Körner, Nüsse, Blätter usw. Unter Speicherorgane im Sinne der Erfindung sind auch Früchte zu verstehen. Früchte sind die Sammelbezeichnung für die den Samen als Nährgewebe umhüllenden Organe der Pflanzen. Dabei ist nicht nur an die eßbaren Früchte, insbesondere an das Obst, aber auch an Hülsenfrüchte, Getreide, Nüsse, Gewürze zu denken, sondern auch an offiziell genutzte Drogen (s. Fructus, Semen). Natürlich kann auch eine Speicherung der Speicherstoffe in der gesamten Pflanze stattfinden.

Bevorzugterweise wird das Leghemoglobin und/oder Hemoglobin knollenspezifisch oder samenspezifisch exprimiert.

Als Pflanzen kommen alle zuvor genannten in Betracht. Bevorzugt werden Kartoffeln, Arabidopsis thaliana, Raps, Sojabohnen, Erdnüsse, Mais, Maniok, Yams, Reis, Sonnenblumen, Roggen, Gerste, Hopfen, Hafer, Hart und Weichweizen, Lupinen, Erbsen, Klee, Rüben, Kohl, Reben usw. wie sie z. B. aus der Verordnung über das Artenverzeichnis zum Saatgutverkehrsgesetz (Blatt für PMZ 1986 S. 3, zuletzt geändert Blatt für PMZ 2002 S. 68) entnehmbar sind, eingesetzt.

Es ist besonders bevorzugt, wenn es sich um knollenproduzierende Pflanzen, insbesondere Kartoffel-Pflanzen oder um samenproduzierende Pflanzen, insbesondere Arabidopsis thaliana, Raps oder Soja handelt.

Die gewebespezifische Expression kann z. B. durch Verwendung eines gewebespezifischen Promotors erreicht werden. Eine solche gewebespezifische Expression ist beispielsweise bekannt aus der US 6,372,961 B1 Spalte 11, Zeilen 44 ff.

Ferner wird eine Nukleotidsequenz gemäß Sequenz Nr. 1 codierend für Leghemoglobin zur Verwendung in einer Pflanze und eine entsprechende Genstruktur oder Vektor sowie deren Verwendung zur Transformierung einer Pflanze mit der Erfindung beschrieben.

Insbesondere fällt unter die Erfindung auch die Verwendung einer Leghemoglobin codierenden Nukleotidsequenz, die mit der Sequenz Nr. 1 zu ca. 70 %, bevorzugt 80%, besonders bevorzugt 85%, 90%, insbesondere 91%, 92%, 93%, 94%, 95%, 96%, 97%, 98% oder 99% identisch ist.

Offenbart sind außerdem Nukleotidsequenzen gemäß Nr. 3 und 5 kodierend für Hemoglobin zur Verwendung in einer Pflanze und eine entsprechende Genstruktur oder -vektor sowie deren Verwendung zur Transformierung einer Pflanze. Insbesondere fallen unter die Erfindung auch die Verwendung von Hemoglobin kodierenden Nukleotidsequenzen, die mit den Sequenzen 3 und 5 zu ca. 70%, bevorzugt 80%, besonders bevorzugt 85%, 90%, insbesondere 91%, 92%, 93%, 94%, 95%, 96%, 97%, 98% oder 99% identisch sind.

Unter Nukleotidsequenz werden alle Nukleotidsequenzen verstanden, die (i) exakt den dargestellten Sequenzen entsprechen; oder (ii) mindestens eine Nukleotidsequenz umfaßen, die innerhalb des Bereichs der Degeneration des genetischen Codes den dargestellten Sequenzen entspricht; oder (iii) mindestens eine Nukleotidsequenz umfasst, die mit einer zur Nukleotidsequenz (i) oder (ii) komplementären Nukleotidsequenz hybridisiert, und gegebenenfalls (iiii) funktionsneutrale Sinnmutationen in (i) umfasst. Dabei bedeutet der Begriff "funktionsneutrale Sinnmutationen" den Austausch chemisch ähnlicher Aminosäuren, wie z. B. Glycin durch Alanin oder Serin durch Threonin.

Auch die den kodierenden Bereichen (Strukturgenen) vorausgehenden (5'-oder upstream) und/oder nachfolgenden (3'-oder downstream) Sequenzbereiche sind eingeschlossen. Insbesondere sind hierin Sequenzbereiche mit regulatorischer Funktion inbegriffen. Sie können die Transkription, die RNA-Stabilität oder das RNA Processing sowie die Translation beeinflussen. Beispiele für regulatorische Sequenzen sind u. a. Promotoren, Enhancer, Operatoren, Teminatoren oder Translationsverstärker.

Unter die jeweiligen Proteine (Leghemoglobine und/oder Hemoglobine) fallen auch Isoformen, die als Proteine mit gleicher oder vergleichbarer Wirkung verstanden werden, die jedoch eine unterschiedliche Primärstruktur aufweisen.

Unter modifizierten Formen sind Proteine zu verstehen, bei denen Änderungen in der Sequenz, beispielsweise am N- und/oder C-Teminus des Polypeptids oder im Bereich konservierter Aminosäuren vorliegen, ohne jedoch die Funktion des Proteins zu beeinträchtigen. Diese Veränderungen können in Form von Aminosäureaustauschen nach bekannten Methoden vorgenommen werden.

Die Erfindung wird unter Bezugnahme auf die folgenden Versuche beispielhaft beschrieben.

**Beispiele**

1. Modellorganismen

Als Modellorganismen für die Experimente wurden Kartoffel und Arabidopsis thaliana eingesetzt. Beide Pflanzenarten repräsentieren ein Mitglied der höheren Pflanzen (Samenpflanzen). Sie können aufgrund des hohen Grades an Homologie ihrer DNA-Sequenzen bzw. Polypeptidsequenzen als Modellpflanzen für andere Pflanzenarten eingesetzt werden.

2. Allgemeine Verfahren

a) Anzucht von Kartoffel oder Arabidopsis Pflanzen

Arabidopsis Pflanzen wurden entweder auf Murashige-Skoog Medium mit 0,5 % Saccharose (Ogas et al., 1997 Science 277:91-94) oder auf Erde gezogen (Focks & Benning, 1998 Plant Physiology 118:91-101). Um einheitliche Keimungs- und Blühzeiten zu erreichen, wurden die Samen nach Ausplattieren bzw. Ausstreuen auf Erde zwei Tage bei 4 °C stratifiziert. Nach der Blüte wurden die Schoten markiert. Entsprechend der Markierungen wurden dann Schoten mit einem Alter von 6-20 Tagen nach der Blüte geerntet. Kartoffelpflanzen wurden nach Dietze et al., 1995 (Gene Transfer to Plants, ed. Potrykus und Spangenberg, Springer Lab Manual, Berlin, Heidelberg, 24-29) angezogen.

b) Isolierung von totalRNA und poly-A+ RNA aus Pflanzen

Für die Herstellung von Expressionskonstrukten wird RNA bzw. polyA+ RNA isoliert. RNA wurde aus Schoten von Arabidopis oder aus Knollen von Kartoffel bzw. Wurzeln von Lotus japonicus nach folgender Vorschrift isoliert: Pflanzenmaterial im Alter von 6 - 40 Tage wurde geerntet und in flüssigem Stickstoff schockgefroren. Das Material wurde vor der weiteren Verwendung bei -80 °C gelagert. 75 mg des Materials wurden im gekühlten Mörser zu einem feinen Pulver gemahlen und mit 200 $\mu$L des Lysis-Puffers aus dem Ambion RNAqueos-Kit versetzt. Die Isolierung der totalen RNA wurde dann nach Herstellerangaben durchgeführt. Die RNA wurde mit 50 $\mu$L Elutionspuffer (Ambion) eluiert und die Konzentration durch Absorption einer 1.100 verdünnten Lösung am Photometer (Eppendorf) bei 260 nm bestimmt. 40ug/ml RNA entspricht dabei einer Absorption von 1. Die RNA-Lösungen wurden mit RNAse freiem Wasser auf eine Konzentration von 1 $\mu$g/$\mu$L eingestellt. Die Konzentrationen wurden durch Agarosegelelektrophorese überprüft.

Zur Isolierung von polyA+ RNA wurde oligo(dT)-Zellulose von Amersham Pharmacia nach Herstellerangaben verwendet. RNA bzw. polyA+ RNA wurde bei -70 °C gelagert.

3. Konstruktion der cDNA-Bank

Zur Konstruktion der cDNA-Bank aus Lotus japonicus- bzw. Arabidopsis thaliana-RNA wurde die Erststrangsynthese unter Verwendung von Reverser Transkriptase aus Maus-Leukämie-Virus (Clontech) und Oligo-d(T)-Primern, die Zweitstrangsynthese durch Inkubation mit DNA-Polymerase I, Klenow-Enzym und RNAse H-Spaltung bei 12 °C (2 Std.), 16 °C (1 Std.) und 22 °C (1 Std.) erzielt. Die Reaktion wurde durch Inkubation bei 65 °C (10 min) gestoppt und anschließend auf Eis überführt. Doppelsträngige DNA-Moleküle wurde mit T4-DNA-Polymerase (Roche, Mannheim) bei 37 °C (30 min) mit glatten Enden versehen. Die Nukleotide wurden durch Phenol/Chloroform-Extraktion und Sephadex-G50-Zentrifugiersäulen entfernt. EcoRI/NotI-Adapter (Pharmacia, Freiburg, Deutschland) wurden mittels T4-DNA-Ligase (Roche, 12°C, über Nacht) an die cDNA-Enden ligiert, mit NotI nachgeschnitten und durch Inkubation mit Polynukleotidkinase (Roche, 37 °C, 30 min) phosphoryliert. Dieses Gemisch wurde der Trennung auf einem Low-Melting-Agarose-Gel unterworfen. DNA-Moleküle über 200 Basenpaaren wurden aus dem Gel eluiert, Phenol-extrahiert, auf Elutip-D-Säulen (Schleicher und Schüll, Dassel, Deutschland) konzentriert und in den Klonierungsvektor pSPORT1 (Invitrogen, Karlsruhe) ligiert, wobei Material des Herstellers verwendet und seine Anweisungen befolgt wurden.

4. DNA-Sequenzierung und Computeranalyse

cDNA-Banken, wie unter Punkt 2 beschrieben, wurden zur DNA-Sequenzierung nach Standardverfahren, insbesondere durch das Kettenterminationsverfahren unter Verwendung des ABI PRISM Big Dye Terminator Cycle Sequencing Ready Reaction-Kit (Perkin-Elmer, Weiterstadt, Deutschland), verwendet. Die Sequenzierung zufälliger, vereinzelter Klone wurde anschließend an die präparative Plasmidgewinnung aus cDNA-Banken über in vivo-Massenexcision und Retransformation von DH5$\alpha$ auf Agarplatten durchgeführt (Einzelheiten zu Material und Protokoll von Stratagene, Amsterdam, Niederlande). Plasmid-DNA wurde aus über Nacht gezüchteten E. coli-Kulturen, die in Luria-Brühe mit Ampicillin (siehe Sambrook et al. (1989) (Cold Spring Harbor Laboratory Press: ISBN 0-87969-309-6)) gezüchtet worden waren, an einem Qiagen-DNA-Präparations-Roboter (Qiagen, Hilden) nach den Protokollen des Herstellers präpariert. Die Sequenzen wurden unter Verwendung des Standard-Softwarepakets EST-MAX, das kommerziell von Bio-Max (München, Deutschland) geliefert wird, prozessiert und annotiert. Durch Nutzung von Vergleichsalgorithmen und unter Verwendung einer Suchsequenz wurde mithilfe des BLAST-Programms nach homologen Genen gesucht (Altschul et al. (1997) "Gapped BLAST and PSI-BLAST: a new generation of protein database search programs", Nucleic Acids Res. 25:3389-3402).

5. Herstellung der Expressionskonstrukte für die knollenspezifische Expression in Kartoffel bzw. die samenspezifische Expression in Arabidopsis:

a) Knollenspezifische Expression:

Das Leghemoglobin-Gen Lljlb wurde aus dem Vektor pSportl mit Notl und Kpnl geschnitten, wobei die Notl Schnittstelle durch Inkubation mit Klenow-Enzym aufgefüllt wurde. Für die Ligation in den Vektor pART33 wurde dieser mit BamHI verdaut, dann die Schnittstelle durch Inkubation mit Klenow-Enzym aufgefüllt und anschliessend mit Kpnl verdaut. pART33 enthält bereits den B33-Promoter (1459 bp; Liu XJ, Prat S, Willmitzer L, Frommer WB (1990) cis regulatory elements directing tuber-specific and sucrose-inducible expression of a chimeric class I patatin promoter/GUS-gene fusion. Mol Gen Genet. 223(3):401-6) sowie den OCS-Terminator (766 bp) für die knollenspezifische Expression. Das gesamte Konstrukt wurde mit Notl aus pART33 geschnitten und in den Vektor für die Pflanzentransformation pART27 einkloniert.

b) Samenspezifische Expression:

Das Leghemoglobin-Gen Lljlb wurde aus dem Vektor pART33 in den Vektor pBINUSP umkloniert. Zu diesem Zweck wurde das Konstrukt pART33-LegHb zunächst mit Asp718I verdaut und die Überhänge mit Klenow-Fragment aufgefüllt. Anschließend wurde das linearisierte Konstrukt mit Xbal-verdaut und das so gewonnene Gen über Nacht bei 4 °C in den Xbal/Smalgeschnittenen pBINUSP ligiert.

Das Hemoglobin-Gen1 AtHb1 wurde aus dem Vektor pART33 in den Vektor pBINUSP umkloniert. Zu diesem Zweck wurde das Konstrukt pART33-AtHb1 zunächst mit Asp7181 verdaut und die Überhänge mit Klenow-Fragment aufgefüllt. Anschließend wurde das linearisierte Konstrukt mit Xbal-verdaut und das so gewonnene Gen über Nacht bei 4 °C in den Xbal/Smalgeschnittenen pBINUSP ligiert.

Das Hemoglobin-Gen2 AtHb2 wurde aus dem Vektor pART33 in den Vektor pBINUSP umkloniert. Zu diesem Zweck wurde das Konstrukt pART33-AtHb2 zunächst mit Asp718I verdaut und die Überhänge mit Klenow-Fragment aufgefüllt. Anschließend wurde das linearisierte Konstrukt mit Xbal-verdaut und das so gewonnene Gen über Nacht bei 4 °C in den Xbal/Smalgeschnittenen pBINUSP ligiert.

6. Plasmide für die Pflanzentransformation

Zur Pflanzentransformation können binäre Vektoren, wie pBIN verwendet werden (Bevan, M., Nucleic Acids Res. 12 (1984), 8711-8721). Die Konstruktion der binären Vektoren kann durch Ligation der cDNA in Sense-oder Antisense-Orientierung in T-DNA erfolgen. 5' der cDNA aktiviert ein Pflanzenpromotor die Transkription der cDNA. Eine Polyadenylierungssequenz befindet sich 3' von der cDNA.

Die gewebespezifische Expression läßt sich unter Verwendung eines gewebespezifischen Promotors erzielen. Beispielsweise kann die samenspezifische Expression erreicht werden, indem der Napin- oder der LeB4- oder der USP-Promotor 5' der cDNA einkloniert wird. Auch jedes andere samenspezifische Promotorelement kann verwendet werden. Zur konstitutiven Expression in der ganzen Pflanzen läßt sich der CaMV-35S-Promotor verwenden.

7. Transformation von Agrobacterium und Pflanzentransformation

Die Agrobacterium-vermittelte Pflanzentransformation kann zum Beispiel unter Verwendung des GV3101-(pMP90-) (Koncz und Schell, Mol. Gen. Genet. 204 (1986) 383-396) oder LBA4404- (Clontech) Agrobacterium tumefaciens-Stamms durchgeführt werden. Die Transformation des Bakteriums kann durch dem Fachmann bekannte Standard-Transformationstechniken durchgeführt werden (Deblaere et al., Nucl. Acids. Tes. 13 (1984), 4777-4788).

Die Agrobacterium-vermittelte Pflanzentransformation selbst kann unter Verwendung von dem Fachmann ebenfalls bekannte Standard-Transformations- und Regenerationstechniken durchgeführt werden (Gelvin, Stanton B., Schilperooft, Robert A., Plant Molecular Biology Manual, 2. Aufl., Dordrecht: Kluwer Academic Publ., 1995, in Sect., Ringbuc Zentrale Signatur: BT11-P ISBN 0-7923-2731-4; Glick, Bernard R., Thompson, John E., Methods in Plant Molecular Biology and Biotechnology, Boca Raton: CRC Press, 1993, 360 S., ISBN 0-8493-5164-2).

Die Transformation mittels Agrobacterium von Arabisopsis thaliana wurde nach der Methode von Bechthold et al., 1993 (C.R. Acad. Sci. Ser. III Sci. Vie., 316, 1194-1199) durchgeführt. Die Methode wurde dahingegen modifiziert, dass auf die Vakuuminfiltration verzichtet wurde.

Für die Transformation wurden *Arabidopsis thaliana* Col0 Samen auf befeuchteter Erde ausgesät, zwei Tage bei 4 °C stratifiziert und 4-6 Wochen unter Kurztagbedingungen (8 h Licht, 21 °C) angezogen. Die Pflanzen wurden dann zur Blühinduktion in Langtagbedingungen (16 h Licht, 21 °C) überführt. Nach etwa 10 Tagen ist die Infloreszenz (Blütenstand) gross genug für das Eintauchen. Die Influoreszenz wird in eine *Agrobacterium* Suspension mit ½ MS Lösung (Murashige und Skoog, s.u.) pH 5,7, 5 % Saccharose, 44 $\mu$M Benzylaminopurin (Sigma) und 0,03 % Silwet L-77 (Lehle Seeds, USA) getaucht. Die optische Dichte der *Agrobacterium* Suspension bei 600 nm sollte 0,8 betragen. Die Bakterien werden zuvor im YEB Medium (0,5 % Bactotrypton, 0,5 % Bactopeptone, 0,1 % Hefeextrakt, 0,5 % Saccharose und 2mM $MgCl_2$) angezogen.

Nach Eintauchen der Pflanzen wurden diese für etwa drei Wochen weiter befeuchtet. Dann wurde die Bewässerung eingestellt und die Pflanzen trockneten für die Samengewinnung ab. Die erhaltenen Samen wurden auf Platten mit ½ MS Lösung, pH 5,7, 50 $\mu$g Kanamycin, 250 $\mu$g Timenten und 0,8 % Agar ausgelegt. Resistente Keimlinge wurden vereinzelt und auf Erde pikiert.

Die Transformation von Kartoffel (Solanum tuberosum L.) erfolgte nach der Methode von Dietze et al. in Gene transfer to plants (eds. I. Potrykus and G. Spangenberg, Springer Lab Manual, 1995, S. 24-29). Als Ausgangsmaterial wurde die Sorte Desiree verwendet.

Zur Transformation wurden fünf bis sechs Blätter einer sterilen Sprossen-Kultur der Kartoffelvarietät Desiree vorsichtig in 10 ml einer 2 MS Lösung (Standard MS Medium nach Murashige and Skoog (1962) A revised medium for rapid growth and bioassays with tobacco tissue cultures. Physiol. Plant., 15, 473-497.) mit zusätzlich 2 % Saccharose equilibriert. Aus den Blättern werden vertikal zur Mittelrippe 1 - 2 mm lange Stücke herausgeschnitten und in frisches 2 MS Medium gegeben. Zu dem frischen Medium werden 50 $\mu$l einer Agrobacterium-Lösung zugegeben, die mit dem T-Plasmid pART27-JpLeg transformiert wurden. Die Agrobacterium-Lösung wird über die Blattstücke verteilt und die Platten werden für zwei Tage im Dunkeln bei 22 - 24 °C inkubiert. Nach zwei Tagen werden die Blattstücke auf CIM Medium (MS Medium mit 1,5 % Glucose, 5 mg/l NAA, 0,1 mg/l BAP, 250 mg/l Claforin und 50 mg/l Kanamycin oder Hygromycin) umgesetzt und 7 Tage inkubiert. Dann werden die Blattstücke auf SIM Medium (MS Medium mit 2 mg/l Zeatin, 0,02 mg/l NAA, 0,02 mg/l GA3, 250 mg/l Claforin und 50 mg/l Kanamycin oder Hygromycin) umgesetzt. Nach 1 - 2 Wochen können dann die entstandenen transgenen Sprosse (1 - 1,5 cm lang) abgeschnitten und auf RIM Medium (MS Medium mit 250 mg/l Claforin) umgesetzt werden. Nach 3 - 4 Wochen haben die Sprossen Blätter und Wurzel ausgebildet und die Pflanzen können auf Erde pikiert werden.

Entsprechend dieser Methode können 2 - 3 unabhängige transgene Linien pro eingesetztes Blatt erhalten werden.

Die alternativ mögliche Pflanzentransformation unter Verwendung von Teilchenbeschuß, Polyethylenglycol-vermittelter DNA-Aufnahme oder über die Siliziumcarbonatfaser-Technik ist beispielsweise beschrieben von Freeling und Walbot "The maize handbook" (1993) ISBN 3-540-97826-7, Springer Verlag New York).

8. Untersuchung der Expression des Leghemoglobins und Hemoglobins in der transformierten Pflanze

Die Aktivität des Leghemoglobins in den transformierten Pflanzen wurde auf der Transkriptionsebene gemessen.

Ein geeignetes Verfahren zur Bestimmung der Menge an Transkription des Gens (ein Hinweis auf die Menge an RNA, die für die Translation des Genproduktes zur Verfügung steht) ist die Durchführung eines Northern-Blots wie unten ausgeführt (als Bezugsstelle siehe Ausubel et al. (1988) Current Protocols in Molecular Biology, Wiley: New York, oder den oben erwähnten Beispielteil), wobei ein Primer, der so gestaltet ist, daß er an das Leghemoglobin bindet, mit einer nachweisbaren Markierung (gewöhnlich radioaktiv oder chemilumineszent) markiert wird, so daß, wenn die Gesamt-RNA einer Kultur des Organismus extrahiert, auf einem Gel aufgetrennt, auf eine stabile Matrix transferiert und mit dieser Sonde inkubiert wird, die Bindung und das Ausmaß der Bindung der Sonde das Vorliegen und auch die Menge der mRNA für dieses Gen (Leghemoglobin bzw. Hemoglobin) anzeigt. Diese Information zeigt den Grad der Transkription des transformierten Gens an. Zelluläre Gesamt-RNA kann aus Zellen, Geweben oder Organen mit mehreren Verfahren, die alle im Fachgebiet bekannt sind, wie zum Beispiel das von Bormann, E. R., et al. (1992) Mol. Microbiol. 6:317-326 beschriebene, präpariert werden.

Northern-Hybridisierung:

Für die RNA-Hybridisierung wurden 20 $\mu$g Gesamt-RNA oder 1 $\mu$g poly(A)[+]-RNA mittels Gelelektrophorese in Agarosegelen mit einer Stärke von 1,25 % unter Verwendung von Formaldehyd, wie beschrieben in Amasino (1986, Anal. Biochem. 152, 304) aufgetrennt, mittels Kapillaranziehung unter Verwendung von 10 x SSC auf positiv geladene Nylonmembranen (Hybond N+, Amersham, Braunschweig) übertragen, mittels UV-Licht immobilisiert und 3 Stunden bei 68 °C unter Verwendung von Hybridisierungspuffer (10 % Dextransulfat Gew./Vol., 1 M NaCl, 1 % SDS, 100 mg Heringssperma-DNA) vorhybridisiert. Die Markierung der DNA-Sonde mit dem Highprime DNA Labeling-Kit (Roche, Mannheim, Deutschland) erfolgte während der Vorhybridisierung unter Verwendung von alpha-$^{32}$P-dCTP (Amersham Pharmacia, Braunschweig, Deutschland). Die Hybridisierung wurde nach Zugabe der markierten DNA-Sonde im gleichen Puffer bei 68 °C über Nacht durchgeführt. Die Waschschritte wurden zweimal für 15 min unter Verwendung von 2 X SSC und zweimal für 30 min unter Verwendung von 1 X SSC, 1 % SDS, bei 68 °C durchgeführt. Die Exposition der verschlossenen Filter wurde bei -70 °C für einen Zeitraum von 1 bis 14 Tagen durchgeführt.

9. Analyse der Auswirkung des rekombinanten Leghemoglobins auf die Produktion des gewünschten Produktes

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder Kohlenhydrate) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).
Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.
Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.
Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).
Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100 °C erhitzt, auf Eis abgekühlt und erneut zentrifugiert, gefolgt von Extraktion in 0,5 M Schwefelsäure in Methanol mit 2 % Dimethoxypropan für 1 Std. bei 90 °C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170 °C und 240 °C für 20 min und 5 min bei 240 °C unterworfen. Die Identität der erhaltenen Fettsäuremethylester muss unter Verwendung von Standards, die aus kommerziellen Quellen erhältlich sind (d.h. Sigma), definiert werden.

10. Analyse der Expression von Lotus-Leghemoglobin, Arabidopsis-Hemoglobin1, Arabidopsis-Hemoglobin2 in reifenden Samen von Arabidopsis.

Zur Überprüfung der Expression der Transgene Hemoglobin1 bzw. Hemoglobin 2 aus Arabidopsis thaliana (AtHb1 bzw. AtHb2) sowie Leghemoglobin aus Lotus japonicus (LjLegHb) wurden Northern Blot-Analysen durchgeführt. Hierzu wurde gesamt RNA aus reifenden Samen transgener und Wildtyp-Pflanzen isoliert, elektrophoretisch aufgetrennt und die Transkripte mit den entsprechenden Digoxigenin-markierten Sonden detektiert. Für jede Linie wurden 2-4 unabhängige Analysen durchgeführt. Abbildung 1 zeigt beispielhaft die Ergebnisse der Expressionsanalysen mit reifenden T2 Samen transgener Arabidopsis-Linien, die entweder Lotus-Hemoglobin (LjLegHb), Arabidopsis-Hemoglobin1 (AtHb1) oder Arabidosis-Hemoglobin2 (AtHb2) exprimieren. In fast allen analysierten Linien wird das transformierte Gen transkribiert, da die entsprechende mRNA nachgewiesen werden kann. In den Kontrollen kann hingegen kein Transkript nachgewiesen werden.

11. Analyse des Ölgehalts von transformierten Lotus-Legehemoglobin und Arabidopsis-Hemoglobine exprimierenden Arabidopsis-Pflanzen

Die Bestimmung der Ölgehalte in den Samen transgener Arabidopsis-Pflanzen, die Lotus-Leghemoglobin, Arabidopsis-Hemoglobin1 oder Arabidopsis Hemoglobin2 exprimieren, erfolgt indirekt über die Quantifizierung der Gesamtfettsäuren. Hierzu wurde folgendes Protokoll angewendet:

Die Extraktion der Lipide aus den Samen erfolgt nach der Methode von Blight & Dyer, 1959 Can. J. Biochem. Physiol. 37:911. Für jede Messung werden hierzu 5-10 Samen in 1,2 ml Qiagen-Microtubes (Qiagen, Hilden) überführt, in einer Retsch Schwingmühle (MM300, Firma Retsch (Haan) pulverisiert und die Gesamtlipide durch Zugabe von 500 μl Chloroform/Methanol (2:1, enthält Pentadecansäure (C15) als internen Standard) extrahiert. Nach Zugabe von 500 μl 50 mM Kaliumphosphatpuffer pH 7,5 erfolgt die Phasentrennung. Die organische Phase wird in ein Pyrrex-Röhrchen überführt und bis zur Trockene eingengt. Anschließend erfolgt die Derivatisierung der Gesamtfettsäuren zu Fettsäuremethylestern nach der Methode von Benning & Sommerville, 1992 J. Bacteriol. 174: 6479-6487 durch Zugabe von 1N $H_2SO_4$ in Methanol und 2 % (v/v) Dimethoxypropane für 1 h bei 80 °C. Diese Fettsäuremethylester werden in Petrolether extrahiert und schließlich gaschromatographisch unter Verwendung einer Kapillarsäule (Chrompack, WCOT Fused Silica, CP-Wax-52, 25 mikom, 0,32 mm) analysiert. Die Quantifizierung der Gesamtfettsäuren erfolgt durch Vergleich der Signalflächen der Fettsäuremethylester mit der Signalfläche des internen Standards bekannter Konzentration. Ein weiterer Indikator für den Ölgehalt in Arabidopsis thaliana ist die Menge der Fettsäure Eicosensäure (20: $1^{\Delta 11}$), welche fast ausschließlich in den Speicherlipiden vorkommt.

Nach der Transformation von Arabidopsis thaliana werden transgene Linien über Antibiotikaresistenz selektioniert. Hierzu wurden T1 Samen transformierter Arabidopsis-Pflanzen aus Hygromycin-haltigen Selektionsplatten ausgekeimt. Für die quantitative Bestimmung der Ölgehalte wurden T2 Samen von jeweils 4 unabhängigen transgenen Linien analysiert. Für die einzelnen Linien wurden hierbei 5-9 unabhängige Einzelmessungen durchgeführt und die entsprechenden Mittelwerte gebildet. Die Ergebnisse der Bestimmung der Ölgehalte in reifen T2 Samen transgener Pflanzen, die Lotus-Leghemoglobin exprimieren, sind beispielhaft in der Tabelle 3 und in der Abbildung 2 gezeigt. Gemäß der Erfindung führt die Expression des Lotus-Leghemoglobins in den Linien 4 und 5 zu einer signifikanten Erhöhung des Ölgehaltes um 17,14 % bzw. 17,54 %.

Die Ergebnisse der Bestimmung der Ölgehalte in reifen T2 Samen transgener Pflanzen, die Arabidopis-Hemoglobin1 exprimieren, sind beispielhaft in der Tabelle 4 und in der Abbildung 3 gezeigt. Gemäß der Erfindung führt die Expression des Arabidopsis-Hemoglobins1 in den Linien 35, 13 und 39 zu einer signifikanten Erhöhung des Ölgehaltes um 9,78 %, 12,22 % bzw. 51,16 %.

Die Ergebnisse der Bestimmung der Ölgehalte in reifen T2 Samen transgener Pflanzen, die Arabidopis-Hemoglobin2 exprimieren, sind beispielhaft in der Tabelle 5 und in der Abbildung 4 gezeigt. Gemäß der Erfindung führt die Expression des Arabidopsis-Hemoglobins 2 in den Linien 2, 10 und 11 zu einer signifikanten Erhöhung des Ölgehaltes um 16,25 %, 21,86 % bzw. 23,80 %.

12. Bestimmung des Stärkegehalts in transformierten Lotus-Leghemoglobin exprimierenden Kartoffelpflanzen

Zur Bestimmung des Stärkegehaltes von Knollen der Kartoffelpflanzen wird die Dichtemessung nach Schéele C. von, Svensson, G. and Rasmusson J., Die Bestimmung des Stärkegehalts und der Trockensubstanz der Kartoffel mit Hilfe des spezifischen Gewichts. Landw. Vers Sta. 127: 67-96, 1937 eingesetzt. Anhand der Dichtemessung kann dann durch Umrechnung auf den Stärkegehalt geschlossen werden. Zur Umrechnung wurde folgende Formel angewendet:

Die spezifische Dichte wurde durch Wiegen der Knollen sowohl in der Luft als auch in Wasser bestimmt, wobei x die Masse in Luft und y die Masse in Wasser ist. Die spezifische Dichte ergibt sich dann aus x/(x-y). Desweiteren wurden folgende Beziehungen aus 560 gemessenen Proben gemittelt (Burton W.G. (1989) The Potato. Longman, New York):

$$\% \text{ Trockenmasse} = 24.182 + [211.04 * (\text{sp. Dichte} - 1.0988)]$$

$$\% \text{ Stärke} = 17.546 + [199.07 * (\text{sp. Dichte} - 1.0988)]$$

Für die Messungen der Leghemoglobin exprimierenden Pflanzen und der Kontrollpflanzen zu Vergleichszwekken wurden unterschiedlich große Kartoffelknollen verschiedener transgenen Linien sowie der Kontrollpflanzen eingesetzt. Die unterschiedlichen Größen dienen dazu, den beobachteten Effekt in allen Knollengrößen zu reproduzieren.

Die Aufarbeitung bzw. Gewinnung von Stärke aus den Leghemoglobin-exprimierenden Kartoffeln kann nach dem Fachmann bekannten üblichen verfahren z. B. gemäss den Angaben der US Patentanmeldung 2001/0041199 A1, Seite 4, Beispiel 1, erfolgen.

Für die Messung des Stärkegehaltes in den Knollen der Leghemoglobin exprimierenden Pflanzen sowie der Kontrollpflanzen zu Vergleichszwecken wurden die Pflanzen sowohl im Gewächshaus als auch im Foliengewächshaus angezogen. Die verschiedenen Kultivierungen dienen dazu, den beobachteten Effekt unter verschiedenen Klimabedingungen zu reproduzieren. Für die Messungen wurden unterschiedlich große Kartoffelknollen eingesetzt, um den beobachteten Effekt in allen Knollengrößen zu reproduzieren.

In Tabelle 6 sind beispielhaft die Werte einer transgenen Linie im Vergleich zu Kontrollpflanzen gezeigt, die beide im Gewächshaus kultiviert wurden. Aufgeführt sind jeweils 6 Messungen verschiedener Knollen einer Linie und die resultierenden Mittelwerte und Standardabweichungen. Dabei kann gemäß der Erfindung eine signifikante Erhöhung des Stärkegehalts in der transgenen Linie um 40,77 % nachgewiesen werden. Für weitere Linien werden ähnliche Werte erreicht.

In Abbildung 5 sind die durchschnittlichen Stärkegehalte von vier unabhängigen transgenen Linien im Vergleich zu Kontrollpflanzen gezeigt, die alle Im Zeitraum vom Mai 2003 bis September 2003 im Foliengewächshaus in Golm kultiviert wurden. Die durchschnittlichen Messwerte basieren hierbei auf 334 Knollen (Linie 13), 358 Knollen (Linie 57), 380 Knollen (Linie 45), 384 Knollen (Linie 54) und 151 Knollen (Wildtyp). Gemäß der Erfindung konnte auch unter den im Foliengewächshaus vorherrschenden Klimabedingungen eine signifikante Erhöhung des Stärkegehaltes in den Knollen der Leghemoglobin-exprimierenden transformierten Pflanzen beobachtet werden, so dass die Ergebnisse aus dem Gewächshaus bestätigt werden konnten. Die im Foliengewächshaus während der Anzucht vorherrschenden Temperaturbedingungen sind der Abbildung 6 zu entnehmen.

Tabelle 3. Ölgehalte in T2 Samen transgener Arabidopsis-Linien, die LjLegHb exprimieren, im Vergleich zur Kontrolle. Die Einzelmessungen wurden mit jeweils 5-10 Samen durchgeführt und die entsprechenden Ölgehalte sind als µg Fettsäuren pro Samen angegeben.

| Linie | WT | Linie 1.1.9 | Linie 3 | Linie 5 | Linie 4 |
|---|---|---|---|---|---|
| | 6,39 | 9,86 | 6,46 | 8,43 | 8,64 |
| | 9,38 | 9,79 | 6,87 | 9,04 | 8,99 |
| | 9,38 | 8,98 | 7,89 | 8,91 | 9,18 |
| | 7,48 | 9,72 | 9,45 | 8,77 | 8,43 |
| | 9,38 | 7,78 | 8,16 | | 6,57 |

(fortgesetzt)

| Linie | WT | Linie 1.1.9 | Linie 3 | Linie 5 | Linie 4 |
|---|---|---|---|---|---|
| | 6,87 | 7,54 | 8,30 | | 10,74 |
| | 8,44 | 7,59 | 6,97 | | |
| | 6,03 | 4,31 | | | |
| | 3,93 | | | | |
| Mittelwert | 7,48 | 8,20 | 7,73 | 8,79 | 8,76 |
| Standardabweichung | 1,77 | 1,74 | 0,96 | 0,23 | 1,23 |
| **relative Ölzunahme** | | **9,61** | **3,34** | **17,54** | **17,14** |

**Tabelle 4.** Ölgehalte in T2 Samen transgener Arabidopsis-Linien, die AtHb1 exprimieren, im Vergleich zur Kontrolle. Die Einzelmessungen wurden mit jeweils 5-10 Samen durchgeführt und die entsprechenden Ölgehalte sind als μg Fettsäuren pro Samen

| Linie | WT | Linie 39 | Linie 35 | Linie 17 | Linie 13 |
|---|---|---|---|---|---|
| | 6,39 | 12,17 | 8,13 | 7,96 | 8,02 |
| | 9,38 | 11,02 | 7,48 | 6,73 | 9,04 |
| | 9,38 | 10,54 | 7,82 | 7,45 | 8,16 |
| | 7,48 | 10,13 | 7,89 | 6,05 | 8,09 |
| | 9,38 | 9,93 | 9,72 | | 8,64 |
| | 6, 87 | 13,40 | | | |
| | 8,44 | 11,36 | | | |
| | 6,03 | 11,22 | | | |
| | 3, 93 | 11,97 | | | |
| Mittelwert | 7,48 | 11,30 | 8,21 | 7,05 | 8,39 |
| Standardabweichung | 1,77 | 1,03 | 0,79 | 0,72 | 0,39 |
| **relative Ölzunahme** | | **51,16** | **9,78** | **-5,77** | **12,22** |

**Tabelle 5.** Ölgehalte in T2 Samen transgener Arabidopsis-Linien, die AtHb2 exprimieren, im Vergleich zur Kontrolle. Die Einzelmessungen wurden mit jeweils 5-10 Samen durchgeführt und die entsprechenden Ölgehalte sind als μg Fettsäuren pro Samen angegeben.

| Linie | WT | Linie 10 | Linie 9 | Linie 11 | Linie 2 |
|---|---|---|---|---|---|
| | 6,39 | 8,36 | 9,79 | 8,26 | 8,73 |
| | 9,38 | 8,53 | 8,50 | 9,66 | 8,36 |
| | 9,38 | 9,83 | 9,79 | 8,23 | 9,11 |
| | 7,48 | 9,72 | 9,93 | 8,98 | 8,57 |
| | 9,38 | | 6,69 | 11,16 | |
| | 6,87 | | 3,06 | | |
| | 8,44 | | 7,54 | | |
| | 6,03 | | | | |
| | 3,93 | | | | |
| Mittelwert | 7,48 | 9,11 | 7,90 | 9,26 | 8,69 |
| Standardabweichung | 1,77 | 0,67 | 2,29 | 1,09 | 0,27 |
| **relative Ölzunahme** | | **21,86** | **5,64** | **23,80** | **16,25** |

**Tabelle 6:** Bestimmung der spezifischen Dichte einer transgenen Linie im Vergleich zu einer Kontrollpflanze.

| | Wildtyp | | | | LegHb | | | |
|---|---|---|---|---|---|---|---|---|
| | Luft | Wasser | Wasser/Luft | Dichte | Luft | Wasser | Wasser/Luft | Dichte |
| Knolle1 | 5,4500 | 0,2700 | 0,0500 | 1,0521 | 13,990 | 1,0000 | 0,0710 | 1,0770 |
| Knolle2 | 8,9900 | 0,3300 | 0,0370 | 1,0381 | 15,530 | 1,1000 | 0,0710 | 1,0762 |
| Knolle3 | 25,7900 | 1,4700 | 0,0570 | 1,0604 | 30,280 | 2,2000 | 0,0730 | 1,0783 |
| Knolle4 | 30,7000 | 1,9300 | 0,0630 | 1,0671 | 50,740 | 3,2300 | 0,0640 | 1,0680 |
| Knolle5 | 37,4500 | 1,9900 | 0,0530 | 1,0561 | 59,430 | 4,0600 | 0,0680 | 1,0733 |
| Knolle6 | 80,9300 | 4,5000 | 0,0560 | 1,0589 | 67,120 | 4,3600 | 0,0650 | 1,0695 |
| Mittelwe rt | | | | **1,0555** | | | | **1,0737** |
| STD | | | | 0,0090 | | | | 0,0039 |
| % Stärke | **8,9181** | | | | **12,554** | | | |
| Erhöhung in % | | | | | **40,77** | | | |

**Abbildung 1.** Northern Blot-Analysen mit reifenden Samen transgenei Arabidopsis-Pflanzen die mit Lotus-Leghemoglobin (LjLegHb), Arabidopsis-Hemoglobin1 (AtHb1) oder Arabidopsis-Hemoglobin2 (AtHb2) transformiert worden waren. Die Menge der eingesetzten Gesamt-RNA erfolgt durch Vergleich der Menge an ribosomaler RNA (rRNA).

**Abbildung 2.** Graphische Darstellung der Ölgehalte in T2 Samen transgener Arabidopsis-Linien, die LjLegHb exprimieren, im Vergleich zur Kontrolle. Aufgeführt sind einerseits die Werte aus 4-9 Einzelmessungen mit jeweils 5-10 Samen (•) und andererseits die resultierenden Mittelwerte mit den dazugehörigen Standardabweichungen (⊙).

**Abbildung 3.** Graphische Darstellung der Ölgehalte in T2 Samen transgener Arabidopsis-Linien, die AtHb1 exprimieren, im Vergleich zur Kontrolle. Aufgeführt sind einerseits die Werte aus 4-9 Einzelmessungen mit jeweils 5-10 Samen (●) und andererseits die resultierenden Mittelwerte mit den dazugehörigen Standardabweichungen (o).

**Abbildung 4.** Graphische Darstellung der Ölgehalte in T2 Samen transgener Arabidopsis-Linien, die AtHb2 exprimieren, im Vergleich zur Kontrolle. Aufgeführt sind einerseits die Werte aus 4-9 Einzelmessungen mit jeweils 5-10 Samen (●) und andererseits die resultierenden Mittelwerte mit den dazugehörigen Standardabweichungen (o).

**Abbildung 5.** Stärkegehalt von Leghemoglobin exprimierenden Kartoffel-Knollen im Vergleich zum Wildtyp. Pflanzen wurden im Foliengewächshaus in Golm im Sommer 2003 gezogen, und abgereifte Knollen geerntet. Der Stärkegehalt wurde durch Bestimmung der Dichte (specific gravity) der Knollen gemessen. Die Daten basieren jeweils auf 334 Knollen (Linie 13), 358 Knollen (Linie 57), 380 Knollen (Linie 45), 384 Knollen (Linie 54) und 151 Knollen (Wildtyp).

**Abbildung 6.** Temperaturmessungen im Foliengewächshaus, in dem die transgenen Kartoffelpflanzen, die das Leghemoglobin aus Lotus japonicus überexprimierten, angezogen wurden. Angegeben sind jeweils die Temperaturmaxima und –minima.

**Patentansprüche**

1. Verfahren zur Erhöhung des Öl-Gehalts in Pflanzen, **dadurch gekennzeichnet, dass** Pflanzen mit einem Vektor enthaltend ein Leghemoglobin -oder Hemoglobin-Gen und einen gewebespezifischen Promotor transformiert werden und das eingebrachte Leghemoglobin oder Hemoglobin samenspezifisch überexprimiert wird.

2. Verfahren nach Anspruch 1, **dadurch gekennzeichnet, dass** die Pflanzen transformiert werden mit einem Vektor enthaltend eine Nukleotidsequenz Nr. 1 codierend für ein Leghemoglobin oder eine Nukleotidsequenz Nr. 3 und/ oder 5 codierend für Hemoglobin oder eine Leghemoglobin- oder Hemoglobin kodierende Nukleotidsequenzen, die mit einer der Sequenzen Nr. 1, 3 und/oder 5 zu 70 % identisch sind.

**3.** Verfahren nach einem der Ansprüche 1 bis 2, **dadurch gekennzeichnet, dass** das Leghemoglobin und Hemoglobin aus Pflanzen aus der Gruppe bestehend aus *Arabidopsis thaliana*, *Lupinus luteus*, *Glycine max*, *Medicago sativa*, *Medicago trunculata*, *Phaseolus vulgaris*, *Vicia faba*, *Pisum sativum*, *Vigna unguiculata*, *Lotus japonicus*, *Psophocarpus tetragonolobus*, *Sesbania rostrata*, *Casuarina glauca* und *Canvalaria lineata* ausgewählt wird.

**4.** Verfahren nach einem der Ansprüche 1 bis 2, **dadurch gekennzeichnet, dass** monokotyle Kulturpflanzen, insbesondere der Art Gramineae transformiert werden.

**5.** Verfahren nach einem der vorhergehenden Ansprüche 1 bis 4, **dadurch gekennzeichnet, dass** dikotyledone Kulturpflanzen, insbesondere der Familie Asteraceae, Brassicacea, Compositae, Cruciferae, Cucurbitaceae, Leguminosae, Rubiaceae, Solanaceae, Sterculiaceae, Theaceae oder Umbelliferae transformiert werden.

**6.** Verfahren nach einem der vorhergehenden Ansprüche 1 bis 5, **dadurch gekennzeichnet, dass** Arabidopsis thaliana, Soja oder Raps transformiert werden.

**7.** Verwendung einer Pflanze mit samenspezifischer Überexpression von Leghemoglobin oder Hemoglobin aufgrund einer Transformation mit einem Vektor enthaltend ein Leghemoglobin -oder Hemoglobin-Gen und einen gewebespezifischen Promotor zur Produktion von Öl.

## Claims

**1.** A method for increasing the oil content in plants, which comprises transforming plants with a vector comprising a leghemoglobin or hemogloblin gene and a tissue-specific promotor and overexpressing the introduced leghemoglobin or hemoglobin in a seed-specific manner.

**2.** The method according to claim 1, wherein the plants are transformed with a vector comprising a nucleotide sequence No. 1 coding for a leghemoglobin, or a nucleotide sequence No. 3 and/or 5 coding for hemoglobin, or a leghemoglobin - or hemoglobin-encoding nucleotide sequences which have 70% identity with any of the sequences No. 1, 3 and/or 5.

**3.** The method according to either of claims 1 and 2, wherein the leghemoglobin and hemoglobin is selected from plants of the group consisting of *Arabidopsis thaliana*, *Lupinus luteus*, *Glycine max*, *Medicago sativa*, *Medicago trunculata, Phaseolus vulgaris*, *Vicia faba*, *Pisum sativum*, *Vigna unguiculata*, *Lotus japonicus*, *Psophocarpus tetragonolobus*, *Sesbania rostrata*, *Casuarina glauca* and *Canvalaria lineata*.

**4.** The method according to either of claims 1 and 2, wherein monocotyledonous crop plants, in particular of the species Gramineae, are transformed.

**5.** The method according to any of the preceding claims 1 to 4, wherein dicotyledonous crop plants, in particular from the family Asteraceae, Brassicacea, Compositae, Cruciferae, Cucurbitaceae, Leguminosae, Rubiaceae, Solanaceae, Sterculiaceae, Theaceae or Umbelliferae are transformed.

**6.** The method according to any of the preceding claims 1 to 5, wherein Arabidopsis thaliana, soybean or oilseed rape are transformed.

**7.** The use of a plant having seed-specific overexpression of leghemoglobin or hemoglobin owing to a transformation with a vector comprising a leghemoglobin or hemoglobin gene and a tissue-specific promotor for the production of oil.

## Revendications

**1.** Procédé pour augmenter la teneur en huile de plantes, **caractérisé en ce qu'**on transforme des plantes avec un vecteur contenant un gène de la leghémoglobine ou de l'hémoglobine et un promoteur spécifique de tissu, et on surexprime d'une manière spécifique de séquence la leghémoglobine ou l'hémoglobine introduite.

**2.** Procédé selon la revendication 1, **caractérisé en ce que** les plantes sont transformées avec un vecteur contenant une séquence nucléotidique n° 1 codant pour une leghémoglobine, ou une séquence nucléotidique n° 3 et/ou 5 codant pour l'hémoglobine, ou une séquence nucléotidique codant pour la leghémoglobine ou l'hémoglobine, qui a une identité allant jusqu'à 70 % avec l'une des séquences n° 1, 3 et/ou 5.

3. Procédé selon l'une des revendications 1 à 2, **caractérisé en ce que** la leghémoglobine et l'hémoglobine sont sélectionnées à partir de plantes du groupe consistant en *Arabidopsis thaliana*, *Lupinus luteus, Glycine max*, *Medicago sativa*, *Medicago trunculata*, *Phaseolus vulgaris*, *Vicia faba*, *Pisum sativum*, *Vigna unguiculata*, *Lotus japonicus*, *Psophocarpus tetragonolobus*, *Sesbania rostrata*, *Casuarina glauca* et *Canvalaria lineata*.

4. Procédé selon l'une des revendications 1 à 2, **caractérisé en ce que** ce sont des plantes de culture monocotylédones, en particulier de l'espèce Gramineae, qui sont transformées.

5. Procédé selon l'une des revendications précédentes 1 à 4, **caractérisé en ce que** ce sont des plantes de culture dicotylédones, en particulier de la famille des Asteraceae, des Brassicaceae, des Compositae, des Cruciferae, des Cucurbitaceae, des Leguminosae, des Rubiaceae, des Solanaceae, des Sterculiaceae, des Theaceae ou des Umbelliferae qui sont transformées.

6. Procédé selon l'une des revendications 1 à 5, **caractérisé en ce que** c'est Arabidopsis thaliana, le soja ou le colza, qui sont transformés.

7. Utilisation d'une plante présentant une surexpression spécifique de semence de leghémoglobine ou d'hémoglobine, se fondant sur une transformation avec un vecteur contenant un gène de la leghémoglobine ou de l'hémoglobine et un promoteur spécifique de tissu, pour production d'huile.

**EP 1 578 977 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6372961 B
- WO 9812913 A
- WO 0000597 A
- US 6372961 B1
- US 20010041199 A1
- DE 10260707

**In der Beschreibung aufgeführte Nicht-Patentliteratur**

- **Quelle.** CD Römpp Chemie Lexikon. Georg Thieme Verlag, 1995
- Reservestoffe. Quelle: Römpp Lexikon Chemie. Georg Thieme Verlag, 1999
- Biochemistry & Molecular Biology of the Plant. American Society of Plant Physiologists, 2000
- **Millar et al.** *Trends Plant Sci,* 2000, vol. 5, 95-101
- Römpp Chemie Lexikon. Georg Thieme Verlag, 1999
- **Ogas et al.** *Science,* 1997, vol. 277, 91-94
- **Focks ; Benning.** *Plant Physiology,* 1998, vol. 118, 91-101
- **Dietze et al.** Gene Transfer to Plants. Springer Lab Manual, 1995, 24-29
- **Altschul et al.** Gapped BLAST and PSI-BLAST: a new generation of protein database search programs. *Nucleic Acids Res.,* 1997, vol. 25, 3389-3402
- **Liu XJ ; Prat S ; Willmitzer L ; Frommer WB.** cis regulatory elements directing tuber-specific and sucrose-inducible expression of a chimeric class I patatin promoter/GUS-gene fusion. *Mol Gen Genet.,* 1990, vol. 223 (3), 401-6
- **Bevan, M.** *Nucleic Acids Res.,* 1984, vol. 12, 8711-8721
- **Koncz ; Schell.** *Mol. Gen. Genet.,* 1986, vol. 204, 383-396
- **Deblaere et al.** *Nucl. Acids. Tes.,* 1984, vol. 13, 4777-4788
- **Gelvin, Stanton B. ; Schilperooft, Robert A.** Plant Molecular Biology Manual. Kluwer Academic Publ, 1995
- **Glick, Bernard R. ; Thompson, John E.** Methods in Plant Molecular Biology and Biotechnology. CRC Press, 1993, 360
- **Bechthold et al.** *C.R. Acad. Sci. Ser. III Sci. Vie.,* 1993, vol. 316, 1194-1199
- **Dietze et al.** Gene transfer to plants. Springer Lab Manual, 1995, 24-29
- **Murashige ; Skoog.** A revised medium for rapid growth and bioassays with tobacco tissue cultures. *Physiol. Plant.,* 1962, vol. 15, 473-497
- **Freeling ; Walbot.** The maize handbook. Springer Verlag, 1993
- **Ausubel et al.** Current Protocols in Molecular Biology. Wiley, 1988
- **Bormann, E. R. et al.** *Mol. Microbiol.,* 1992, vol. 6, 317-326
- **Amasino.** *Anal. Biochem.,* 1986, vol. 152, 304
- **Ullman.** Encyclopedia of Industrial Chemistry. VCH, 1985, vol. A2, 89-90443-613
- **Fallon, A. et al.** Applications of HPLC in Biochemistry. *Laboratory Techniques in Biochemistry and Molecular Biology,* 1987, vol. 17
- Product recovery and purification. **Rehm et al.** Biotechnology. VCH, 1993, vol. 3, 469-714
- **Belter, P.A. et al.** Bioseparations: downstream processing for Biotechnology. John Wiley and Sons, 1988
- **Kennedy, J.F. ; Cabral, J.M.S.** Recovery processes for biological Materials. John Wiley and Sons, 1992
- Biochemical Separations. **Shaeiwitz, J.A. ; Henry, J.D.** Ullmann's Encyclopedia of Industrial Chemistry. VCH, 1988, vol. B3, 1-27
- **Dechow, F.J.** Separation and purification techniques in biotechnology. Noyes Publications, 1989
- **Cahoon et al.** *Proc. Natl. Acad. Sci. USA,* 1999, vol. 96 (22), 12935-12940
- **Browse et al.** *Analytic Biochemistry,* 1986, vol. 152, 141-145
- **Christie, William W.** Advances in Lipid Methodology. Oily Press
- **Christie, William W.** Gas Chromatography and Lipids. A Practical Guide. Oily Press, 1989, 307
- Progress in Lipid Research. Pergamon Press, 1952
- Applied Microbial Physiology; A Practical Approach. IRL Press, 103-129131-163165-192

- Advances on Lipid Methodology. Christie, Oily Press, 1997, 119-169
- Gaschromatographie-Massenspektrometrie-Verfahren. *Lipide,* 1998, vol. 33, 343-353
- **Blight ; Dyer.** *Can. J. Biochem. Physiol.,* 1959, vol. 37, 911
- **Benning ; Sommerville.** *J. Bacteriol.,* 1992, vol. 174, 6479-6487
- **Schéele C. von ; Svensson, G. ; Rasmusson J.** Die Bestimmung des Stärkegehalts und der Trockensubstanz der Kartoffel mit Hilfe des spezifischen Gewichts. *Landw. Vers Sta.,* 1937, vol. 127, 67-96
- **Burton W.G.** The Potato. Longman, 1989